# EUROPEAN PATENT SPECIFICATION

(11) **EP 3 936 633 B1**
(45) Date of publication and mention of the grant of the patent: **06.09.2023**
(21) Application number: 19917924.3
(22) Date of filing: 13.11.2019
(51) Int. Cl.: C23C 8/02, C23C 8/10, C23C 8/42, C23C 8/80, C22C 16/00, A61L 31/12, C22F 1/18

(54) **TREATMENT METHOD FOR ZIRCONIUM ALLOY AND APPLICATION**
BEHANDLUNGSVERFAHREN FÜR ZIRKONIUMLEGIERUNG UND VERWENDUNG
PROCÉDÉ DE TRAITEMENT POUR ALLIAGE DE ZIRCONIUM ET APPLICATION

(30) Priority: 07.03.2019 CN 201910172951
(43) Date of publication of application: 12.01.2022
(73) Proprietor: Suzhou Microport Orthorecon Co., Ltd., Suzhou, Jiangsu 215000 (CN)
(72) Inventor: SHEN, Yu, Suzhou, Jiangsu 215000 (CN); YU, Tianbai, Suzhou, Jiangsu 215000 (CN); WENG, Chih-Hsin, Suzhou, Jiangsu 215000 (CN)
(74) Representative: Patentship Patentanwaltsgesellschaft mbH
(86) International application number: PCT/CN2019/117752
(87) International publication number: WO 2020/177382

(56) References cited:
- CN-A- 104 818 409
- CN-A- 109 069 690
- CN-A- 109 706 421
- CN-A- 109 825 797
- US-A- 4 507 184
- US-A1- 2007 137 734
- US-A1- 2007 251 604
- US-A1- 2013 230 640
- US-B2- 8 361 381
- Huang Yongchang; Zhang Jianqi: "Passage; Modern Material Corrosion and Protection" In: Huang Yongchang; Zhang Jianqi: "Modern Material Corrosion and Protection", 30 September 2012 (2012-09-30), XP009523020, ISBN: 978-7-313-08277-0 * pp. 79 and 80 *

## Description

### TECHNICAL FIELD

The present application relates to the field of materials for medical implants, in particular, to a treatment method for a zirconium alloy and application thereof.

### BACKGROUND

Materials for use in medical implants are required to have high strength, corrosion resistance and histocompatibility. This makes only a part of metal alloys to be desirable materials meeting above requirements, such as 316L stainless steel, cobalt-chromium-molybdenum alloys, titanium alloys, as well as zirconium alloys that have been recognized in recent years as the most suitable materials for manufacturing bearing and non-bearing prostheses.

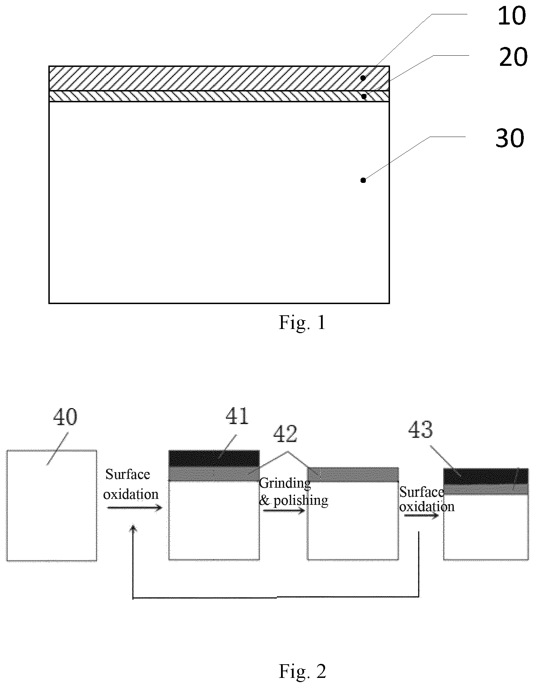

In general, a zirconium alloy has a relatively soft surface with a hardness that range from 1.5 GPa to 3 GPa, making it vulnerable to abrasion by harder third-body particles and thus owing a poor wear-resisting property. Traditionally, the surface hardness of the zirconium alloy is usually improved by surface oxidization or surface nitriding in prior art. The principle of surface oxidization is to form an oxide ceramic surface on the zirconium alloy. The surface hardness for zirconium oxide may be up to 12 GPa. Fig. 1 shows a structural model of an oxide ceramic surface. As shown, the oxide ceramic surface layer usually has a thickness of 5-6 µm and the oxygen-rich diffusion layer having a thickness of about 1.5-2 µm is present at the interface between the oxide ceramic surface layer and the metal substrate. Such structure enables to offer a hard ceramic surface as well as keep the good plasticity of the zirconium alloy substrate, thereby resulting in improved surface resistance to wear and scratching and avoiding the risk of brittle cracking arising from the use of ceramic materials in manufacturing prostheses.

U.S. Patents US2987352 and US3615885 each describe the approach of heating the zirconium alloy in air to form an oxide ceramic surface layer thereon. The surface of a medical implant treated by such approach owns excellent resistances to wear, scratching and brittle cracking, thereby showing the good effect of such approach. At present, the zirconium alloy material used in practical manufacturing is originated from the zirconium alloy material used in nuclear industry, which is surface oxidized to form a oxide ceramic surface layer with a color of dark blue. The oxide ceramic surface layer with a color of dark blue has a high compactness and few cracks. However, the final products are very expensive due to the very high price of raw material.

U.S. Patent Application, US 2007/0137734 A1, discloses a medical implant of zirconium or zirconium alloy comprising a thick diffusion hardened zone and a ceramic layer, as well as a method of treating the medical implant in vacuum or in an inert gas environment at an elevated temperature. Such a method is able to generate a specific diffusion hardened zone, but is unable to solve problems of the large amount of micro-cracks and the low compactness of oxide ceramic layer in the zirconium alloy.

U.S. Patent Application, US 2007/0251604 A1, discloses a method of reworking a zirconium or zirconium alloy comprising an oxidation and/or nitridation layer on its surface, comprising treating the zirconium or zirconium alloy under reduced pressure or in an inert gas environment at an elevated temperature until the oxidation and/or nitridation layer is substantially removed from the surface. Such a method is directed to reclaim the rejected components due to the failure of the oxidation treatment, and does not teach to solve problems of the large amount of micro-cracks and the low compactness of oxide ceramic layer in the zirconium alloy.

U.S. Patent Application, US 2013/230640 A1, discloses a method of making a layered ceramic medical implant comprising the steps of: forming said medical implant of zirconium or zirconium alloy; treating said implant in the presence of ceramic-forming species at temperature of 500 to 1000 °C for greater than 2 minutes; and, thereafter treating said implant under a reactive gas at a temperature of 500 to 1000°C. Such a method is able to achieve the surface hardness and the specific characteristics of ceramic layers, but is unable to solve problems of the large amount of micro-cracks and the low compactness of oxide ceramic layer in the zirconium alloy.

U.S. Patent 4,507,184 discloses a method for solving the problem of the deposition of stain in the recesses to cause loss of beautiful appearance on the matted surface of an article for personal ornament made of titanium, zirconium or an alloy thereof formed by honing or barrel finishing. The method comprises the steps of (a) forming a first matted surface with microscopically fine protrusions and recesses on the article by honing or barrel finishing, (b) forming a hardened layer on the matted surface by nitriding, carbonizing, bonding or oxidizing, (c) partially removing the hardened layer covering the protrusions on the matted surface by a first electrolytic or chemical polishing, (d) forming a second matted surface on the article by honing or barrel finishing, and (e) partially removing the surface layer at the protrusions by a second electrolytic or chemical polishing to smoothen the surface leaving a sandy appearance. However, such a method does not aim to solve the problems of the large amount of micro-cracks and the low compactness of oxide ceramic layer in the zirconium alloy.If the zirconium alloy material used in general industries and having a much low price is adopted to produce the medical implant having an oxide ceramic surface layer, they would produce the oxide ceramic surface layer with a color of grey white even treated with a same processing. It has been found in practice that the oxide ceramic layer with a color of grey white is bad in compactness and bonding strength and some of them are even found with shedding of oxide particles from the surface during the preparation of cross-sectional samples. Since the exfoliated oxide particles from the surface have very high hardness, such type of oxide ceramic surface layer cannot improve wear resistance of the zirconium alloys, but accelerate the rate of wear due to the generation of a huge amount of exfoliated oxide particles, which results in its inapplicability for the manufacture of medical implants.

Therefore, it would be desirable to develop a method capable of replacing the expensive zirconium alloy material used in the nuclear industry with the zirconium alloy material used in general industries to lower the cost under the premise that the performance of the produced oxide ceramic layer meets the requirements.

### SUMMARY

The inventors have found through painstaking researches that a low compactness of an oxide ceramic layer for the zirconium alloy is attributable to an excessive content of hafnium element in the zirconium alloy material, which causes a large amount of micro-cracks to be produced in the oxide ceramic layer. Present application provides a method for reducing the content of hafnium element on the surface of the zirconium alloy and the application thereof to solve the problems of the large amount of micro-cracks and the low compactness of oxide ceramic layer for the zirconium alloy.

To solve the above technical problems, technical solution of present application provides:
a method of treating a zirconium alloy, comprising: a step of performing a surface layer oxidation and removal treatment on the zirconium alloy, where the surface layer oxidation and removal treatment comprises: performing an oxidation treatment on a surface layer of the zirconium alloy to obtain an oxide surface layer; and performing a removal treatment to the oxide surface layer to expose a metal substrate, wherein the zirconium alloy has an initial content of hafnium element ranging from 0.5 wt% to 8 wt%, and the oxidation treatment is conducted at a temperature of 500 °C to 700 °C and a treatment time of 0.5 h to 10 h.

Optionally, the oxide surface layer is removed by grinding, fine machining, mechanical polishing, vibratory polishing or any combination thereof.

Optionally, a thickness of the oxide surface layer removed in the removal treatment ranges from 1µm to 20µm.

Optionally, the thickness of the oxide surface layer removed in the removal treatment ranges from 3µm to 12µm.

Optionally, the method further comprises repeating the step of performing a surface layer oxidation and removal treatment for 1 to 5 times.

The present application also provides a method for producing an oxide ceramic layer on a surface of a zirconium alloy, comprising treating the zirconium alloy with the above described method; and performing an oxidation treatment on a surface of the exposed metal substrate.

Optionally, the oxide ceramic layer has a content of hafnium element ranging from 0.3 wt% to 6 wt%.

The present application also provides a material for use in medical implants comprising a metal substrate, an oxygen-rich diffusion layer and an oxide ceramic layer, the metal substrate made of a zirconium alloy, where a content of hafnium element in the metal substrate is higher than a content of hafnium element in the oxide ceramic layer, the content of hafnium element in the metal substrate ranging from 0.5 wt% to 8 wt%, the content of hafnium element in the oxide ceramic layer ranging from 0.3 wt% to 6 wt%.

The technical solution of present application is able to lower the contents of hafnium oxides in the oxide ceramic surface layer of the zirconium alloy having a high content of hafnium element, so as to solve the problem of micro-cracks in the oxide ceramic surface layer of the high-hafnium-content zirconium alloy, and thus offer the oxide ceramic layer improved abrasive resistance, hardness and damage resistance. Moreover, this method is simple and has a low cost. In addition, compared with the method using expensive low-hafnium-content zirconium alloy as the raw material, the method provided in present application is able to achieve the oxide ceramic layer with comparable performance as well as dramatically reduce the cost of raw material, thereby making it more competitive in the marketplace. In particular, the method provided in present application is suitable for the surface treatment of a material for use in joint prostheses.

### BRIEF DESCRIPTION OF THE DRAWINGS

Fig. 1 schematically illustrates a cross-sectional structure of an oxide ceramic surface layer formed on a zirconium alloy in prior art.
Fig. 2 is a flowchart graphically illustrating a process for reducing the content of hafnium oxide in an oxide ceramic surface layer on a zirconium alloy according to a particular embodiment.
Fig. 3 is a Scanning Electron Microscope (SEM) image showing the cross section of an oxide ceramic layer of Sample 1 according to Embodiment 1.
Fig. 4 is a Scanning Electron Microscope (SEM) image showing the cross section of an oxide ceramic layer of Sample 2 according to Embodiment 1.
Fig. 5 is a Scanning Electron Microscope (SEM) image showing the cross section of an oxide ceramic layer of Sample 3 according to Embodiment 1.
Fig. 6 shows hardness curve graphs of the oxide ceramic layers of Samples 1 to 3 according to Embodiment 1.
Fig. 7 shows an indentation photo of Sample 2 according to Embodiment 1 created by a Rockwell diamond indenter under a load of 60 kg.
Fig. 8 shows an indentation photo of Sample 3 according to Embodiment 1 created by a Rockwell diamond indenter under a load of 60 kg.
Fig. 9 is a metallurgical microscope image showing the cross-sectional morphology of an oxide ceramic layer of Sample 4 according to Embodiment 2.
Fig. 10 is a metallurgical microscope image showing the cross-sectional morphology of an oxide ceramic layer of Sample 5 according to Embodiment 2.
Fig. 11 is a metallurgical microscope image showing the cross-sectional morphology of an oxide ceramic layer of Sample 6 according to Embodiment 2.
Fig. 12 shows hardness curve graphs of the oxide ceramic layers of Samples 4 to 6 according to Embodiment 2.

In the figures:
10, oxide ceramic surface layer; 20, oxygen-rich diffusion layer; 30, metal substrate; 40, first surface layer; 41, first oxide surface layer; 42, second surface layer; and 43, second oxide surface layer.

### DETAILED DESCRIPTION

The inventors have found through investigations and researches that the zirconium alloy material inevitably contains a certain quantity of hafnium element as an impurity because the two elements coexist in nature. In zirconium ores, the ratio of the weight percentage of hafnium element to the weight percentage of zirconium element generally ranges from 1.5% to 3.0%. Due to the very close properties of hafnium and zirconium elements, it is difficult to separate hafnium element from the zirconium element. Existing techniques for separating hafnium from zirconium are all costly and tend to cause environmental pollution. In common industrial applications, as the presence of hafnium does not affect the mechanical and chemical properties of alloys, removal of hafnium element generally is unnecessary. However, in the nuclear industry, as the hafnium element has a large thermal neutron absorption area, the presence of hafnium impedes the use of the zirconium alloys as a cladding material in nuclear industry. Therefore, it is necessary to separate hafnium from the zirconium alloy to produce the zirconium alloy with a very low content (<0.005%) of hafnium element.

Through detections of oxide ceramic surface layers, it has been found that the surface and interior of the oxide ceramic layer produced from zirconium alloy material for use in general industries have numerous micro-cracks. The contents of various major elements in the oxide ceramic layer have been measured on the surface and at different cross-sectional depths of the oxide ceramic layer. It has been found from the measurement results that hafnium oxide is widely distributed within the oxide ceramic layer.

From the above facts, the inventors speculate that the primary reason for the decreased compactness of the oxide ceramic layer is the formation of micro-cracks in the oxide ceramic layer caused by the hafnium oxide in the oxide ceramic layer.

The contents of various major elements have been measured on the surfaces of the metal substrate of the oxidized zirconium alloy, oxygen-rich diffusion layer and oxide ceramic surface layer and at different cross-sectional depths of the oxide ceramic surface layer. It has been found from the measurement results that although hafnium oxide is widely distributed within the oxide ceramic layer, the content of hafnium element in the zirconium alloy substrates near the oxide ceramic layer goes down.

The principle for this phenomenon may be as follow. In oxidation, the Gibbs free energy for the formation of hafnium dioxide is -1087.2kJ/mol, and the Gibbs free energy for the formation of zirconium dioxide is -1038.7 kJ/mol. Since the Gibbs free energy for the formation of hafnium dioxide is lower than that for the formation of zirconium dioxide, the hafnium element is easier to combine with oxygen than the zirconium element. The hafnium atom preferentially binds to oxygen in oxidation and thus enriches on the surface. This results in a decreased content of hafnium element in the zirconium alloy substrates near the oxide ceramic layer.

Accordingly, after the first surface oxidation, the oxide surface layer (i.e., the oxide ceramic layer and oxygen-rich diffusion layer) with enriched hafnium element is ground and polished to the near-surface substrate having a decreased content of hafnium element. Then, the second surface oxidation is performed on such surface (i.e., the near-surface substrate) to obtain the oxide ceramic surface layer with reduced content of hafnium element, thereby reducing micro-cracks within oxide ceramic surface layer and improving quality of the oxide ceramic surface layer. Although such method can only reduce the content of hafnium on the surface, it is totally suitable as the material for use in medical implant as the presence of hafnium inside the alloy does not affect the performance of the medical implants. Besides, compared with the method using zirconium alloy for the nuclear industry as raw material, this method is advantageous in easier availability of raw material and much lower cost.

Specifically, present application prepares a material for use in medical implants by the following method. Such material includes a metal substrate 30, an oxygen-rich diffusion layer 20 and an oxide ceramic layer 10 that are arranged from inside to outside. The metal substrate 30 is a zirconium alloy that is not surface oxidized. The oxide ceramic layer 10 is a layer in which oxygen element is present essentially in the form of the oxide. The oxygen-rich diffusion layer 20 is a layer in which the content of oxygen is higher than the content of oxygen in metal substrate and the oxygen element is present essentially in the form of solute atoms. Hafnium is present in the metal substrate 30 at an amount of 0.5 wt% to 8 wt%. The oxide ceramic layer 10 has a lower content of hafnium than the metal substrate 30, which is 0.3 wt %-6 wt%, preferably 0.3 wt%-2 wt%, more preferably 0.3 wt%-1 wt%, e.g., 0.4 wt%, 0.5 wt%, 0.7 wt% or 0.9 wt%.

As shown in Fig. 2, steps of the method for preparing a material for use in medical implants are as follows:
(1) Performing a surface oxidation treatment on the first surface layer 40 of a zirconium alloy metal substrate 30 containing a hafnium content of 0.5 wt% to 8 wt%. The surface oxidation treatment may be performed in air or in any other oxygen-containing atmosphere. Alternatively, it may also be performed using a vapor, a water bath or a salt bath. The surface oxidation process may be performed at a oxidation temperature of 500 °C to 700 °C, preferably 550 °C to 600 °C, for a treatment time of 0.5-10 h, preferably 4-6 h. The first oxide surface layer 41 enriched with hafnium therein is formed on the surface of the metal substrate 30 after the surface oxidation treatment. The first oxide surface layer 41 consists of an oxygen-rich diffusion layer 20 and an oxide ceramic layer 10.
(2) Removing the first oxide surface layer 41 enriched with hafnium by means of an approach selected from the group consisting of grinding, fine machining, mechanical polishing, vibratory polishing and any combination thereof with a removal thickness of 1-20 µm, preferably 3-12 µm, so as to expose the second surface layer 42 with reduced content of hafnium element. Preferably, the removal thickness is selected as the thickness that is able to exactly remove the first oxide surface layer 41. That is, the removal thickness is selected as the thickness that is able to exactly remove the oxygen-rich diffusion layer 20 and the oxide ceramic layer 10 together with partial metal substrate. The thickness of the oxygen-rich diffusion layer 20 and the oxide ceramic layer 10 each may be determined from a cross-section measurement of the material. This is because both the oxygen-rich diffusion layer 20 and the oxide ceramic layer 10 are distinguishable from a cross section of the material by the naked eye. Alternatively, the thickness may also be speculated based on the used oxidation conditions and previous experience.
(3) Repeating step (1) to perform another surface oxidation treatment to form a second oxide surface layer 43 with a reduced content of hafnium element.
(4) Repeating steps (1) to (3) for one time, or more times, preferably 1-5 times, until the formed oxide ceramic layer has a reduced hafnium content of 0.3 wt% to 6 wt%, preferably 0.3 wt% to 2 wt%, more preferably 0.3 wt% to 1 wt%.

For ease of understanding, the method for reducing the content of hafnium oxide in an oxide ceramic surface layer of a zirconium alloy provided in present application will be described below with reference to several embodiments. It is to be understood that these embodiments are described for the mere purpose of illustration and do not limit the protection scope thereof in any sense.

Unless particularly noted, each material or reagent used in the following embodiments is commercially available, and each process or parameter can be realized by existing technology.

### Embodiment 1

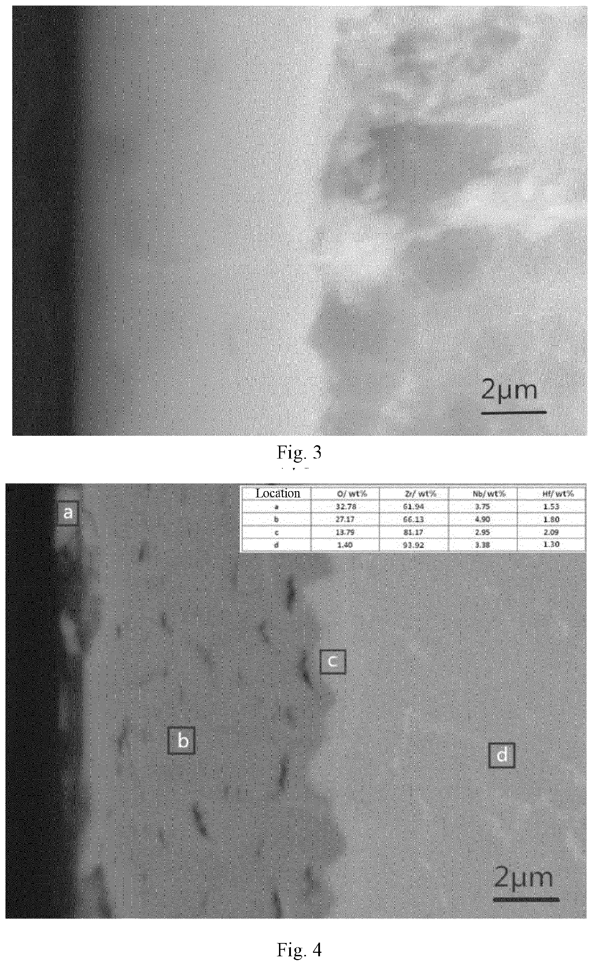

Samples 1 and 2 each with an oxide ceramic surface layer were prepared by heating zirconium alloys with hafnium contents of <0.005% and about 2.26% to 550 °C and maintaining them at the temperature for 6 h in air respectively. Scanning Electron Microscope (SEM) images showing their cross-sectional morphologies were shown in Figs. 3 and 4. In each of the images, there were an oxide ceramic layer on the left, an oxygen-rich diffusion layer in the middle and a metal substrate on the right. As can be seen in Fig. 3, the oxide ceramic surface layer of Sample 1has a high compactness with few defects. However, as shown in Fig. 4, there were numerous micro-cracks in the oxide ceramic surface layer of Sample 2. Elemental analysis results of Sample 2 show that oxide ceramic surface layer (1.53 wt% at location a and 1.80 wt% at location b) and the oxygen-rich diffusion layer (2.09 wt % at location c) have high contents of the hafnium element, while the location of the substrate near the oxide ceramic layer (location d) has a hafnium content (1.3 wt %) remarkably lower than the initial hafnium content (2.26 wt %) of the alloy.

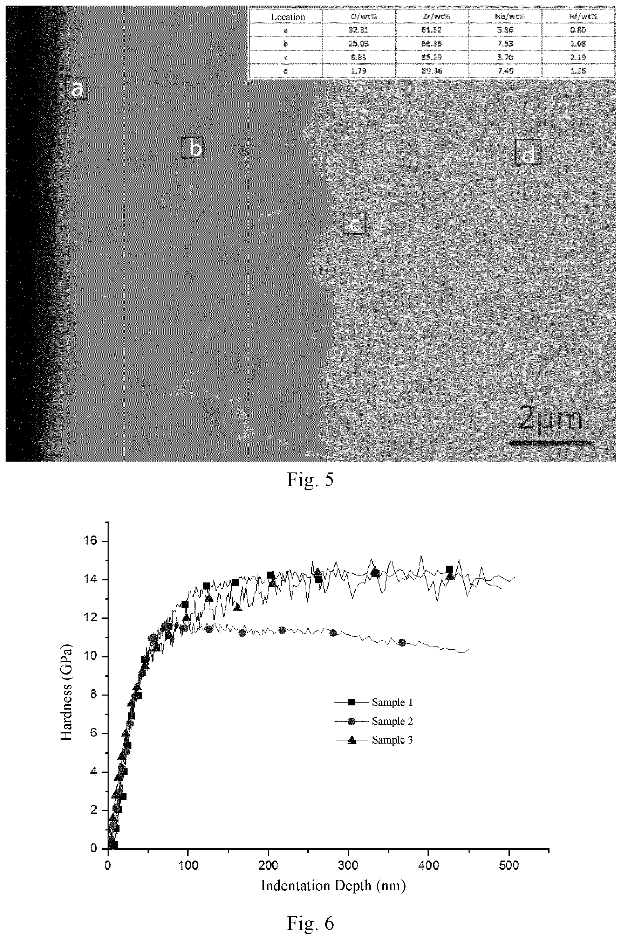

The oxide ceramic surface layer of Sample 2 was removed by means of mechanical grinding, polishing or another approach with a removal thickness of about 10 µm. That is, the oxide ceramic surface layer and the oxygen-rich diffusion layer were removed to expose the surface with a low hafnium content. Subsequently, the surface with a low hafnium content was performed a surface oxidization treatment by heating to 550 °C and maintaining at the temperature for 6 h in air to obtain Sample 3. Fig. 5 shows a cross-sectional morphology and the contents of various elements at different locations of Sample 3. As can be seen in Fig. 5, the oxide ceramic surface layer of Sample 3 obtained by above method exhibits a high compactness without noticeable cracks. Percentages of various major elements at different cross-sectional locations of Samples 2 and 3 measured by a Energy Disperse Spectroscopy (EDS) were listed at upper right corners of Figs. 4 and 5, respectively. The measurements show a greatly reduced content of hafnium element in the oxide ceramic surface layer (locations a and b) of Sample 3. For the surface layer of Sample 2, the percentage of hafnium in the total content of zirconium, niobium and hafnium is about 2.28 wt%, while for the surface layer of Sample 3 obtained by the above method, the percentage of hafnium in the total content of zirconium, niobium and hafnium is about 1.18 wt%.

Fig. 6 shows hardness curve graphs of the oxide ceramic surface layers of Samples 1 to 3. The hardness curve graph is measured by a nanoindentation instrument in a CSM (Continuous Stiffness Measurement) mode with an indentation depth of up to 500 nm and the plotted values were average hardness measured at indentation depths in the range of 200-400 nm. Samples 1 and 3 each exhibit a higher surface hardness value (approx. 14.2 GPa for each) while Sample 2 exhibits a low surface hardness value (approx. 11.1 GPa). The enhanced hardness value demonstrates the improved wear resistance of the material. Thus, this method allows improving wear resistance of the oxide ceramic surface layer formed by the zirconium alloy having a high hafnium content.

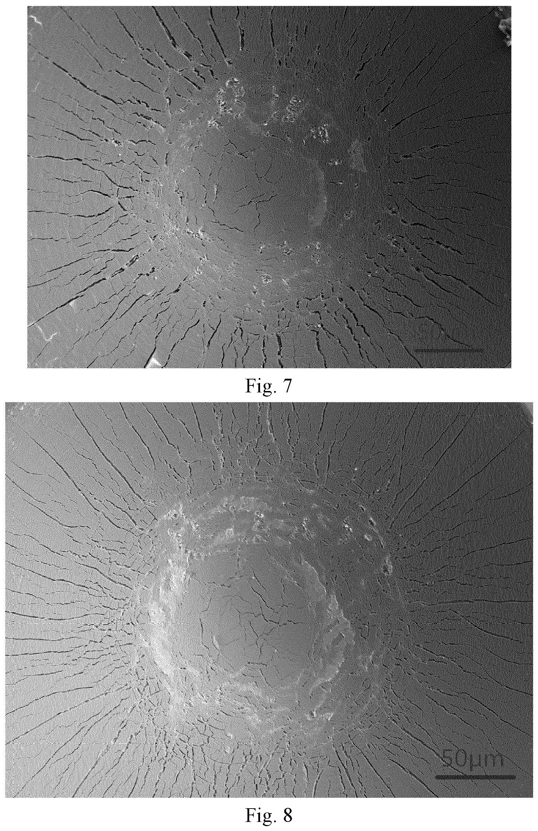

Figs. 7 and 8 show indentations of Samples 2 and 3 created by a Rockwell diamond indenter under a load of 60 kg respectively, to characterize anti-crushing properties of the oxide ceramic surface layers. As can be seen, the indentation created on Sample 2 was deeper and broader than that on Sample 3, demonstrating the ability of the above method to improve the anti-crushing properties of a zirconium alloy with a high content of hafnium element.

### Embodiment 2

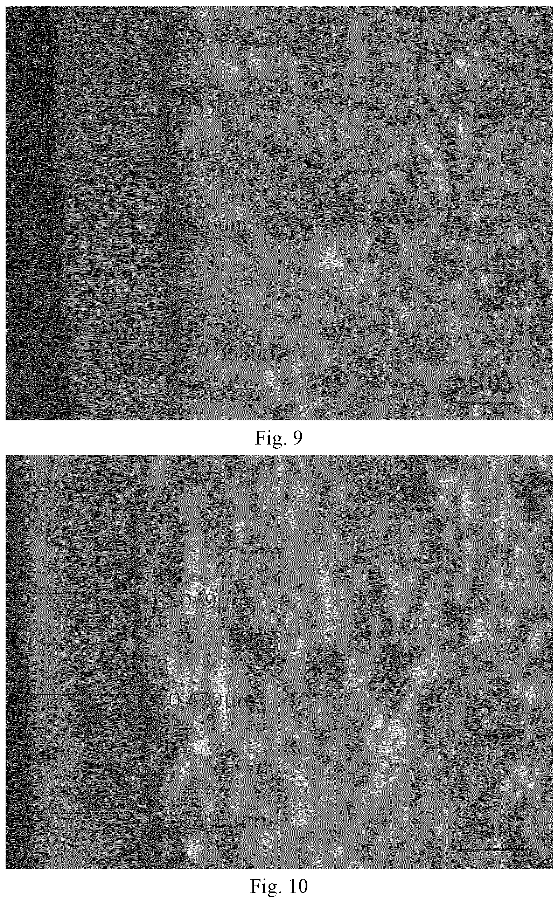

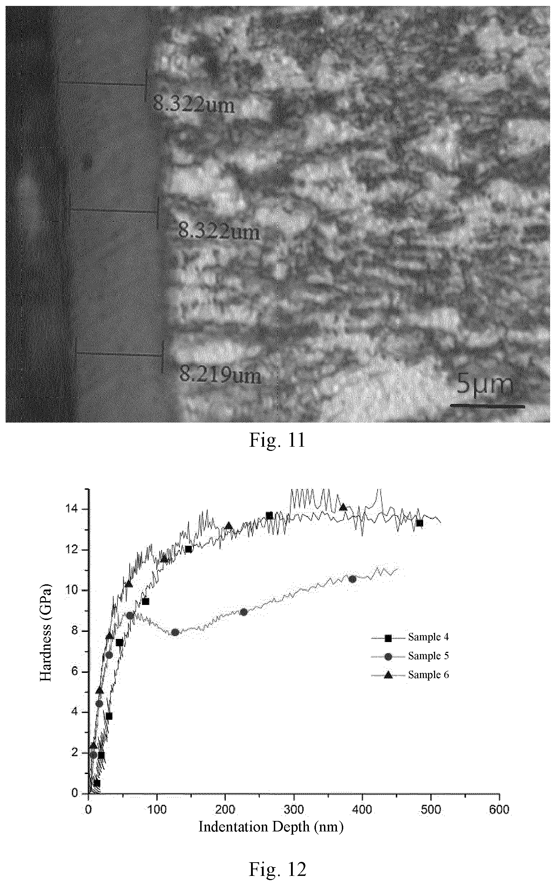

Samples 4 and 5 each with an oxide ceramic surface layer were prepared by heating zirconium alloys with hafnium contents of <0.005% and about 1.8% to 600 °C and maintaining them at the temperature for 4 h in air respectively. Cross-sectional morphologies of Samples 4 and 5 were observed with a metallurgical microscope, and corresponding metallurgical microscope images were shown respectively in Figs. 9 and 10. In each of the images, there were an oxide ceramic layer on the left, an oxygen-rich diffusion layer in the middle and a metal substrate on the right. Compared with Sample 5, Sample 4 has an oxide ceramic surface layer possessing a high interior compactness, a tight bonding to the substrate without any crevice in the interface, as well as a thickness of about 9.66 µm. The Sample 5 has an oxide ceramic layer possessing a relatively large thickness of about 10.51 µm, a low interior compactness as well as numerous micro-cracks, even the tendency of cracking. In addition, the oxide ceramic layer of Sample 5 has a poor bonding to the substrate with distinct crevices. Both the oxide ceramic surface layer and the oxygen-rich diffusion layer of Sample 5 were removed by removing a surface thickness of about 12 µm of the sample through mechanical grinding, polishing or another means. Subsequently, Sample 5 was again heated at 600 °C in air for 4 h to obtain the Sample 6. The metallurgical cross-sectional morphology of Sample 6 was shown in Fig. 11. The morphology of oxide ceramic layer of Sample 6 is similar to that of oxide ceramic layer of Sample 4. Thus, after treated by the method of present application, the oxide ceramic layer for the formed Sample 6 has a thickness of about 8.29µm, and obtains a considerably improved quality, thereby enabling to achieve the low hafnium content similar to that of Sample 4

Fig. 12 shows hardness curve graphs of the oxide ceramic surface layers of Samples 4 to 6. The hardness curve graph is measured by a nanoindentation instrument in a CSM (Continuous Stiffness Measurement) mode with an indentation depth of up to 500 nm and the plotted values were average hardnesses measured at indentation depths in the range of 200-400 nm. Samples 4 and 6 each exhibit a high surface hardness value (approx. 14.0 GPa and 13.6GPa respectively) while Sample 5 exhibits a low surface hardness value (approx. 9.7 GPa). The higher hardness value means the better wear resistance of the material. Thus, this method is able to further improve wear resistance of the oxide ceramic surface layer formed by the zirconium alloy having a high hafnium content.

It is to be noted that, the medical implants as mentioned herein refer to implantable medical instruments that can be placed into surgically created or naturally occurring cavities in human bodies. Examples of the medical implants may include, but are not limited to, surgical implants such as artificial joints, (orthopedic, spinal, cardiovascular and neurosurgical) implants, structural prostheses, dentures and other artificial organs; implants made of metal materials (including stainless steel, cobalt-based alloys, titanium and alloys thereof and shape memory alloys), polymers, high molecular materials, inorganic non-metallic materials, ceramic materials, etc.; implantable instruments such as implantable orthopedic instruments, implantable aesthetic and plastic surgical instrument and materials; implantable appliances such as bones (plates, screws, pins, rods), intra-spinal fixation devices, staplers, patellar concentrators, bone wax, bone repair materials, plastic surgical materials, heart or tissue repair materials, intraocular filling materials, nerve patch, etc.; interventional instruments such as interventional catheters, stents, embolization and other devices; and orthopedic (orthopedic) surgical instruments such as scalpels, drills, scissors, forceps, saws, chisels, files, hooks, needle slickers, active instruments, extremity extension braces, multi-purpose unilateral external fixation devices and other instruments for orthopedic (orthopedic) surgical use.

## Claims

1. A method of treating a zirconium alloy, comprising a step of performing a surface layer oxidation and removal treatment on the zirconium alloy, wherein the surface layer oxidation and removal treatment comprises: performing an oxidation treatment on a surface layer (40, 42) of the zirconium alloy to obtain an oxide surface layer (41, 43); and performing a removal treatment to the oxide surface layer to expose a metal substrate (30), wherein the zirconium alloy has an initial content of hafnium element ranging from 0.5 wt% to 8 wt%, and the oxidation treatment is conducted at a temperature of 500 °C to 700 °C and a treatment time of 0.5 h to 10 h.

2. The method of claim 1, wherein the oxide surface layer (41,43) is removed by means of grinding, fine machining, mechanical polishing, vibratory polishing or any combination thereof.

3. The method of claim 1, wherein a thickness of the oxide surface layer (41,43) removed in the removal treatment ranges from 1µm to 20µm.

4. The method of claim 3, wherein the thickness of the oxide surface layer (41,43) removed in the removal treatment ranges from 3µm to 12µm.

5. The method of claim 1, further comprising repeating the step of performing a surface layer oxidation and removal treatment for 1 to 5 times.

6. A method for producing an oxide ceramic layer (10) on a surface of a zirconium alloy, comprising treating the zirconium alloy with the method of any one of claims 1 to 5; and performing an oxidation treatment on a surface of the exposed metal substrate.

7. The method of claim 6, wherein the oxide ceramic layer (10) has a content of hafnium element ranging from 0.3 wt% to 6 wt%.

8. A material for use in medical implants, prepared by the method of any one of claims 1 to 7 and comprising a metal substrate (30), an oxygen-rich diffusion layer (20) and an oxide ceramic layer (10), the metal substrate (30) made of a zirconium alloy, wherein a content of hafnium element in the metal substrate (30) is higher than a content of hafnium element in the oxide ceramic layer (10), wherein the content of hafnium element in the metal substrate (30) ranges from 0.5 wt% to 8 wt%, and the content of hafnium element in the oxide ceramic layer (10) ranges from 0.3 wt% to 6 wt% .

## Patentansprüche

1. Verfahren zur Behandlung einer Zirkoniumlegierung, umfassend einen Schritt eines Durchführens einer Oberflächenschichtoxidations und -entfernungsbehandlung auf der Zirkoniumlegierung, wobei die Oberflächenschichtoxidations und -entfernungsbehandlung umfasst: Durchführen eine Oxidationsbehandlung auf einer Oberflächenschicht (40, 42) der Zirkoniumlegierung, um eine Oxidoberflächenschicht (41, 43) zu erhalten; und Durchführen einer Entfernungsbehandlung an der Oxidoberflächenschicht, um ein Metallsubstrat (30) freizulegen, wobei die Zirkoniumlegierung einen initialen Gehalt von Hafniumelement in einem Bereich von 0,5 Gew.-% bis 8 Gew.-% aufweist, und wobei die Oxidationsbehandlung bei einer Temperatur von 500 °C bis 700 °C und einer Behandlungszeit von 0,5 h bis 10 h durchgeführt wird.

2. Verfahren nach Anspruch 1, wobei die Oxidoberflächenschicht (41, 43) durch Schleifen, Feinbearbeitung, mechanisches Polieren, Vibrationspolieren oder eine beliebige Kombination davon entfernt wird.

3. Verfahren nach Anspruch 1, wobei eine Dicke der bei der Entfernungsbehandlung entfernten Oxidoberflächenschicht (41, 43) in einem Bereich von 1 µm bis 20 µm ist.

4. Verfahren nach Anspruch 3, wobei die Dicke der bei der Entfernungsbehandlung entfernten Oxidoberflächenschicht (41, 43) in einem Bereich von 3 µm bis 12 µm ist.

5. Verfahren nach Anspruch 1, ferner umfassend ein 1-maliges bis 5-maliges Wiederholen des Schritts des Durchführens einer Oberflächenschichtoxidations- und -entfernungsbehandlung.

6. Verfahren zur Herstellung einer Oxidkeramikschicht (10) auf einer Oberfläche einer Zirkoniumlegierung, umfassend Behandeln der Zirkoniumlegierung mit dem Verfahren nach einem der Ansprüche 1 bis 5; und Durchführen einer Oxidationsbehandlung auf einer Oberfläche des freigelegten Metallsubstrats.

7. Verfahren nach Anspruch 6, wobei die Oxidkeramikschicht (10) einen Gehalt von Hafniumelement in einem Bereich von 0,3 Gew.-% bis 6 Gew.-% aufweist.

8. Material zur Verwendung in medizinischen Implantaten, hergestellt durch das Verfahren nach einem der Ansprüche 1 bis 7 und umfassend ein Metallsubstrat (30), eine sauerstoffreiche Diffusionsschicht (20) und eine Oxidkeramikschicht (10), wobei das Metallsubstrat (30) aus einer Zirkoniumlegierung gemacht ist, wobei ein Gehalt von Hafniumelement in dem Metallsubstrat (30) höher als ein Gehalt von Hafniumelement in der Oxidkeramikschicht (10) ist, wobei der Gehalt von Hafniumelement in dem Metallsubstrat (30) in einem Bereich von 0,5 Gew.-% bis 8 Gew.-% ist, und wobei der Gehalt von Hafniumelement in der Oxidkeramikschicht (10) in einem Bereich von 0,3 Gew.-% bis 6 Gew.-% ist.

## Revendications

1. Procédé de traitement d'un alliage de zirconium, comprenant une étape d' exécution d'un traitement d'oxydation et d'élimination de la couche superficielle sur l' alliage de zirconium, dans lequel le traitement d'oxydation et d'élimination de la couche superficielle comprend : l'exécution un traitement d'oxydation sur une couche superficielle (40, 42) de l'alliage de zirconium pour obtenir une couche superficielle d'oxyde (41, 43); et performant un traitement d'élimination de la couche superficielle d'oxyde pour exposer un substrat métallique (30), dans lequel l' alliage de zirconium a une teneur initiale en élément hafnium allant de 0,5 % en poids à 8 % en poids, et l' oxydation le traitement est effectué à une température de 500 °C à 700 °C et une durée de traitement de 0,5 h à 10 h.

2. Procédé selon la revendication 1, dans lequel la couche superficielle d'oxyde (41, 43) est éliminée au moyen d'un meulage, d'un usinage fin, d'un polissage mécanique, d'un polissage vibratoire ou de toute combinaison de ceux-ci.

3. Procédé selon la revendication 1, dans lequel une épaisseur de la couche superficielle d'oxyde (41, 43) éliminée lors du traitement d'élimination est comprise entre 1 µm et 20 µm.

4. Procédé selon la revendication 3, dans lequel l'épaisseur de la couche superficielle d'oxyde (41, 43) éliminée lors du traitement d'élimination est comprise entre 3 µm et 12 µm.

5. Procédé selon la revendication 1, comprenant en outre la répétition de l'étape d' exécution d'un traitement d'oxydation et d'élimination de la couche de surface 1 à 5 fois.

6. Procédé de production d'une céramique oxyde couche (10) sur une surface d'un alliage de zirconium, comprenant le traitement de l' alliage de zirconium avec le procédé de l'une quelconque des revendications 1 à 5; et effectuer un traitement d' oxydation sur une surface du substrat métallique exposé.

7. Procédé selon la revendication 6, dans lequel la céramique oxydée couche (10) a une teneur en élément hafnium allant de 0,3 % en poids à 6 % en poids.

8. Matériau destiné à être utilisé dans des implants médicaux, préparé par le procédé de l'une quelconque des revendications 1 à 7 et comprenant un substrat métallique (30), une couche de diffusion riche en oxygène (20) et une couche céramique d'oxyde (10), le métal substrat (30) constitué d'un alliage de zirconium, dans lequel une teneur en élément hafnium dans le substrat métallique (30) est supérieure à une teneur en élément hafnium dans la couche céramique d'oxyde (10), dans lequel la teneur en élément hafnium dans le substrat métallique (30) varie de 0,5 % en poids à 8 % en poids, et la teneur en élément hafnium dans la couche céramique d'oxyde (10) varie de 0,3 % en poids à 6 % en poids.
